# EUROPEAN PATENT SPECIFICATION

(11) **EP 1 015 428 B1**
(45) Date of publication and mention of the grant of the patent: **02.05.2003**
(21) Application number: 98946097.7
(22) Date of filing: 17.09.1998
(51) Int. Cl.: C07D 207/416

(54) **SULFO-N-HYDROXY SUCCINIMIDE AND METHOD OF PREPARATION**
SULFO-N-HYDROXYSUCCINIMIDE UND VERFAHREN ZUR IHREN HERSTELLUNG
SULFO-N-HYDROXYSUCCINIMIDE ET PROCEDE D'ELABORATION

(30) Priority: 18.09.1997 US 932851; 18.09.1997 US 933266
(43) Date of publication of application: 05.07.2000
(73) Proprietor: PIERCE CHEMICAL COMPANY, Rockford, IL 61105 (US)
(72) Inventor: BREMMER, Martin, Lee, Rockford, IL 61102 (US); WILKES, Marty, Carey, Rockton, IL 61072 (US)
(74) Representative: Beresford, Keith Denis Lewis
(86) International application number: US9819364
(87) International publication number: WO99014192

(56) References cited:
- EP-A- 0 046 274
- EP-A- 0 247 866
- EP-A- 0 700 933
- EP-A- 0 726 252
- WO-A-89/12624
- DE-A- 4 317 651
- DE-A- 4 336 802
- DE-C- 943 050
- GB-A- 2 054 589
- GB-A- 2 253 850
- US-A- 4 794 189
- CHEMICAL ABSTRACTS, vol. 50, no. 3, 10 February 1956 Columbus, Ohio, US; abstract no. 1803e, D. E. AMES ET AL.: "The Synthesis of some N-hydroxyimides" column 1; XP002090040 & J. CHEM. SOC., - 1955 pages 631-636, London
- CHEMICAL ABSTRACTS, vol. 108, no. 25, 20 June 1988 Columbus, Ohio, US; abstract no. 222062j, ANJANEYULU, P. S. R. ET AL.: "Reactions of N-hydroxysulfosuccinimide active esters" page 611; column 2; XP002090041 & INT. J. PEPT. PROTEIN RES., vol. 30, no. 1, 1987, pages 117-24,

## Description

### 1. Field of the Invention

The present invention relates to a method for the synthesis of sulfo-N-hydroxy succinimide salts, novel reduced-impurity or impurity-free salts, and novel intermediate hydroxamic acid sulfonate salts.

### 2. Background of the Art

Sulfo-N-hydroxy succinimides (often referred to as "Sulfo-NHS" or "S-NHS") including the acid and salt counterparts have a wide range of utility in a number of broad commercial areas, including but not limited to reagents for the manufacture of biotinylation reagents, oil well drilling agents, chemical and biological assay reagents, crosslinking agents for organic biological systems or polymer systems, side chain modifying agents, solubilizing agents, reactants, markers, and the like. The class may be generally represented by the formula below, representing the central nucleus: 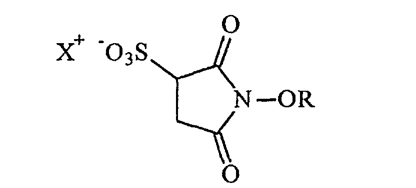 Wherein X⁺ is a cation and R is H or an organic group, or especially any organic group formed from a compound R-OH wherein R-OH is an acid, and the symbolic extraction of OH (the hydroxyl) may leave the group R-, forming an ester with the remainder of the central nucleus. Examples of preferred R-OH compounds are acetic acid, LC-biotin suberic acid, biotin, suberic acid, 4-[N-maleimidomethyl]-cyclohexane-1-carboxylic acid, and the like. The 2- and 3-positions on the central nucleus may also be substituted. The cation may be H', monovalent cations (such as H⁺, Na⁺, Li⁺, K⁺, NH₄⁺,Cs⁺,other inorganic cations, organic cations, etc.), or polyvalent cations (including divalent cations) in which the remaining charge is satisfied by other anions (e.g., halides, nitrates, sulfates, phosphates, etc.) or forms a bis- or tris- configuration with other sulfo-NHS anions. This class of compounds is relatively expensive, mainly because of the expensive synthetic procedures which must be taken to obtain the product. Existing synthetic procedures must not only use a large number of reagents and involve a large number of synthetic steps, but the procedures involve the use of large volumes of solvents and different solvents which must be stripped after various steps as well as at the end of the procedure. The process cost involved in recapturing and stripping of the solvents is quite significant, and with increasing environmental concerns, the requirements for avoiding release of solvents into the atmosphere have become more strict and therefore more costly.

A typical synthetic process for the synthesis of sulfo-N-hydroxysuccinimide salt is known to follow the following route:
Maleic anhydride: 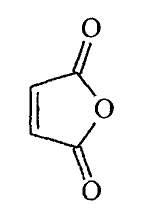 is reacted with furan: 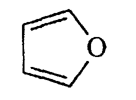 to form a Diels - Alder reaction product:

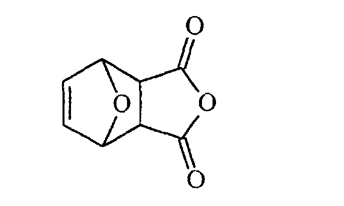

This intermediate product is extremely hazardous and special precautions are required in its handling. Workers must be protectively clothed and may even be required to wear full closure protective gear (e.g., full body suits), including self-contained helmets and at least filters if not self-contained air supplies. The crystals formed are hazardous to the eyes and are easily propelled and carried by air currents. Even removal of protective garments can be hazardous because of clinging crystalline product which can be put into the air by movement of the clothing. The Diels-Alder product is then reacted with hydroxylamine (e.g., hydroxylamine hydrochloride): 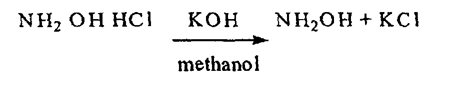 This step is done with potassium hydroxide and methanol (precipitating potassium as potassium chloride) producing an N-hydroxy succinimide adduct:

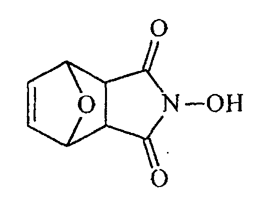

This product is usually washed in toluene and hexane. The N-hydroxy succinimide adduct is then reacted at the hydroxyl group. This reaction is performed by combining the adduct with phenylchloroformate

C₆H₅O₂CCl

(which is a strong lachrymator) in various combinations of triethylamine, dichloromethane, toluene and hexane and sometimes dimethyl formamide to produce the next intermediate product:

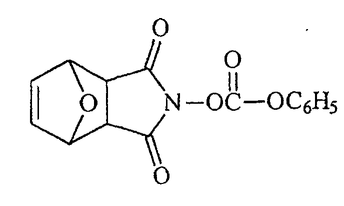

This intermediate product is in turn dissolved in a hydrocarbon solvent, e.g., a non-polar hydrocarbon solvent (e.g., decane) and heated to elevated temperature to form the next intermediate by removal of the protecting group.

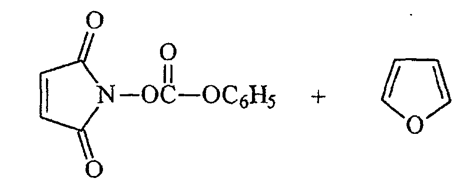

The temperature is elevated to about 170°C, which is above the flashpoint for decane (46°C). The literature also shows the use of nitrobenzene as the solvent in this step. The reaction product tends to be a black, tarry product as result of using this commercially difficult step. t-Butyl catechol may be used as an antioxidant in this step.

This last intermediate is then reacted with sodium metabisulfite in ethanol to form the sodium salt of sulfo-N-hydroxysuccinimide, which is recrystallized from aqueous methanol, isopropanol, and washed with acetone: 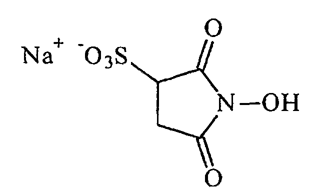 This product is produced in about 95-98% purity as an amorphous solid even after repeated purification, with clear evidence of the succinimide counterpart (the succinimide or hydrogen analog of the hydroxysuccinimide) being present in the final product: 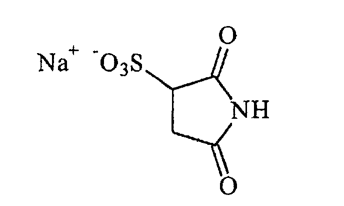 Overall yield of the process from the original maleic anhydride is about 25-28% theoretical, and the complete time of the process is about 50 days. Numerous solvent strips must occur, and a kilogram of product is usually produced in reaction vessels of fifty liters or more.

It therefore can be seen that the entire synthetic route is complex, has toxicity, environmental and hazard concerns throughout, is expensive, and is time consuming. Improved methods of synthesis are clearly desirable.

### SUMMARY OF THE INVENTION

The salt of a sulfonated succinic acid is cyclized (e.g., with a Blanc reaction), then converted to novel sulfonated hydroxamic acids by reaction with hydroxylamine, and the novel hydroxamic acid is then cyclized (e.g., by dehydration) to the sulfo-N-hydroxysuccinimide salt. A process of forming a sulfo-N-hydroxysuccinimide by cyclizing a sulfohydroxamic acid is also described. Alternatively, the present invention describes a novel method for the synthesis of sulfo-N-hydroxy succinimide salts in which commercially available sulfosuccinic acid salt in an aqueous medium is heated in the presence of an alcohol to form a diester of the succinic acid sulfonate. The diester is then reacted with hydroxylamine (e.g., from the hydrochloride salt) (e.g., in an alkaline buffered aqueous environment, e.g., at room temperature) to form the sulfo-N-hydroxy succinimide. This is an extremely simple and direct process that does not produce a significant level of irremovable impurities.

### DETAILED DESCRIPTION OF THE INVENTION

The synthetic procedure of the present invention comprises fewer steps, can be performed in a batch process, requires fewer solvents, and produces novel intermediates and products without similar impurities as compared to processes of previous commercial use. Fewer hazardous materials are synthesized and used, and the process may be performed in a few days (e.g., 2-5 days) as compared to the approximate 50 days used for alternative procedures. Additionally, kilogram product amounts can be produced in a five liter batch process.

One process of the present invention may be described as a synthetic process comprising the steps of:
a) cyclizing a sulfosuccinic acid compound to form a monocyclic first product, and
b) opening the ring of said monocyclic first product,
characterised by opening the ring of said monocyclic first product in the presence of hydroxylamine (e.g. from an acid complex) to form a sulfo-hydroxamic acid.

The process may more specifically comprise cyclizing a sulfo-succinic acid having the central nucleus of: 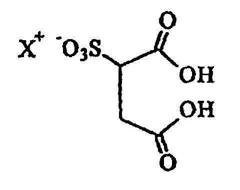 to form a monocyclic first product, and
b) opening the ring of said monocyclic first product in the presence of hydroxylamine acid complex to form a sulfo-hydroxamic acid. The process may be performed, for example, where the sulfo-hydroxamic acid comprises a compound having the central nucleus of:

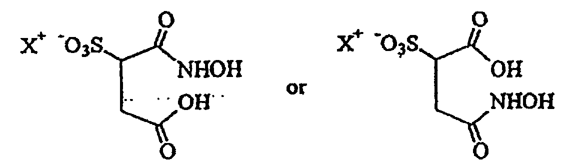

The process may then continue towards the sulfo-N-hydroxysuccinimide wherein the sulfo-hydroxamic acid is then cyclized, as by dehydration, to form a monocyclic sulfo-N-hydroxysuccinimide, wherein the sulfo-N-hydroxysuccinimide comprises the central nucleus of:

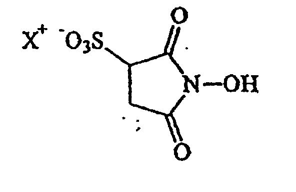

The cyclization (e.g., by dehydration) of the sulfo-hydroxamic acid is itself a novel process which may occur in the presence of methanol, water, acetic acid, acetic anhydride, dicyclohexylcarbodiimide and/or carbonyldiimidazole, as well as any other medium which assists or acts in the dehydration of the hydroxamic acid. The dehydration may be effected merely by leaving the hydroxamic acid in a solution of the additional material, as at room temperature in water, or at slightly elevated temperatures in water. This process may be done where opening a ring of said monocyclic first product in the presence of hydroxylamine acid complex to form a sulfo-hydroxamic acid is done in the presence of excess alcohol to produce a sulfo-hydroxamic acid ester (monoester or diester, depending upon the degree of excess) as a partial product. This ester subsequently performs in essentially the same manner as the acid in conversion to the sulfo-N-hydroxysuccinimide. The alcohol is preferably methanol, but any alcohol or even glycol may be used in this step, since the esterifying moiety is subsequently removed during cyclization or dehydration of the hydroxamic acid. Because of the relatively low number of steps and the reduced amount of residues, waste material and numbers of solvents, the alcohol or glycol is reformed during the dehydration, and this may be readily recovered. This further reduces costs by recycling the alcohol or glycol and avoiding its release into the environment.

Another process of the present invention may be described as a synthetic process for synthesizing a monocyclic sulfo-N-hydroxy succinimide comprising the steps of:
a) esterifying a sulfo-succinic acid to form a diester first product (e.g. a diester sulfosuccinate), and
b) reacting the diester with hydroxylamine hydrochloride (added or formed *in situ*, as by the combination of NaNO₂ and Na₂SO₃), forming the sulfo-N-hydroxysuccinimide.

Another process of the present invention may be described as a synthetic process for synthesizing a monocyclic sulfo-N-hydroxy succinimide comprising the steps of:
a) reacting a sulfosuccinate diester with hydroxylamine (e.g., hydroxylamine hydrochloride, added or formed *in situ*, as by the combination of NaNO₂ and Na₂SO₃), to form the corresponding sulfo-N-hydroxysuccinimide.

It is not certain what the reaction mechanism is in the conversion of the diester to the sulfo-N-hydroxy succinimide, but it is surmised that a sulfohydroxamic acid ester is formed and that this sulfohydroxamic acid is dehydrated/cyclized (e.g., by ester hydrolysis) to form the sulfo-N-hydroxysuccinimide.

This part of the reaction scheme in the process is theoretical as to its mechanism, but the process may continue from the diester to the Sulfo-N-hydroxysuccinimide wherein a sulfo-hydroxamic acid is cyclized, as by ester hydrolysis and/or dehydration, to form a monocyclic sulfo-N-hydroxysuccinimide, wherein the sulfo-N-hydroxy succinimide comprises the general nucleus of: 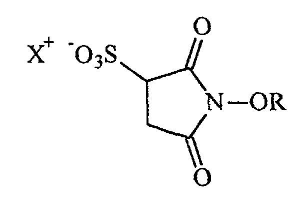 wherein R is as defined as above.

The cyclization, e.g., by dehydration, of the sulfo-hydroxamic acid may occur in the presence of methanol, water, acetic acid, acetic anhydride, dicyclohexylcarbodiimide and/or carbonyldiimidazole, as well as any other medium which assists or acts in the dehydration of the hydroxamic acid. The dehydration may be effected merely by leaving the hydroxamic acid in a solution of the additional material, as at room temperature in water, or at slightly elevated temperatures in water.

The diesters formed during the process of the present invention may be generally described as compounds having the core structure or general nucleus of: 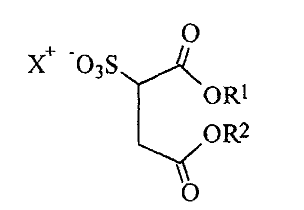 wherein X⁺ is any cation (as described above), R¹ and R² are independently selected from the group consisting of aliphatic and aromatic groups, especially alkyl and aryl groups, and most particularly alkyl groups of 1 to 8 carbon atoms and phenyl groups.

In the practice of the present invention, the alcohol used to form the diester is preferably methanol, but any alcohol (e.g., C1 to C30, preferably C1 to C8, more preferably C1 to C4) or even glycols (e.g., of 2 to thirty carbon atoms) may be used in this step, since the esterifying moiety is subsequently removed during cyclization or dehydration (e.g., of the hydroxamic acid, if formed). Because of the relatively low number of steps and the reduced amount of residues, waste material and numbers of solvents, the alcohol or glycol is reformed during the dehydration, and this may be readily recovered. This further reduces costs by recycling the alcohol or glycol and avoiding its release into the environment.

The sulfo-N-hydroxysuccinimide, as elsewhere indicated herein, is present in the absence of the corresponding sulfosuccinimide (NH or hydrogen compounds) analogs. By further separation and control of enantiomeric components, the sulfo-N-hydroxysuccinimide may have proportions of R and S enantiomers of the sulfo-N-hydroxysuccinimide present, for example, as 100 to 55% or 100 to 50% (or more than 50%, e.g., 51% or more) R or S enantiomers, and may be present as a white, crystalline powder. The sulfo-N-hydroxysuccinimide in any of these forms may be present in the absence of sulfosuccinimide (hydrogen) analogs.

The process may begin with the appropriate sulfo-succinic acid, purchased commercially or formed by the sulfonation of succinic anhydride with sulfur trioxide. The first reagent is a compound having the basic core structure: 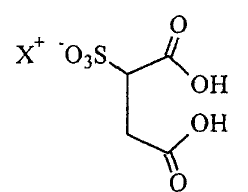 wherein X+ is any cation, preferably a monovalent cation such as H⁺, Na⁺, Li⁺, K⁺, NH₄⁺, etc. Where the term 'core structure' or 'groups' is used, the formula includes any substitution which does not change the actual atoms and bond structure shown. That is, for example with the succinimide and hydroxamic acid, on the unsubstituted portion between or intermediate the point of attachment of the sulfonate group and the carbonyl, any substitution may be present. The term 'having a core' or 'central nucleus' of Formula I therefore includes: 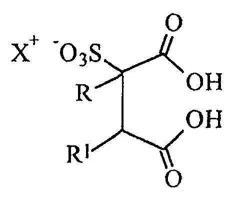 wherein R is H or any other desired substituent. For example, R may be alkyl, alkoxy, halo (I, Cl, Br, F), cyano, alkenyl, aryl (such as phenyl), etc. Likewise for R¹, R¹ may be hydrogen, alkyl, alkoxy, halo (I, Cl, Br, F), cyano, alkenyl, aryl (e.g., phenyl), etc. Where the terminology a 'compound of the formula' is used, that terminology excludes any substitution not specifically included in the description, e.g., allowing only the inherently understood R¹ at the position between the point of attachment of the sulfonate and the carboxy group. Likewise, the terminology of 'a core formula' or 'central nucleus' could prevent any substitution where a double bond was inserted into the backbone of the hydrocarbon chain of the compound.

The initial reagent having the core structure of the formula: 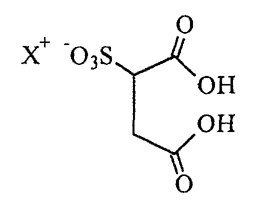 is cyclized. This cyclization reaction may be performed, for example, with the common Blanc reaction, in sodium hydroxide and acetic anhydride. The resulting first intermediate was a central nucleus of the formula: 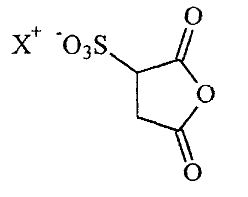 This is a known compound and has a CAS registry number. This first product is then subjected to a ring opening reaction with hydroxylamine (e.g., as an acid adduct, as with hydrochloride) in methanol, producing the novel hydroxamic acid salts:

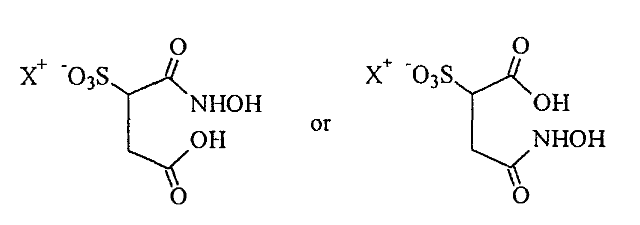

These are novel compounds which have not been reported in the literature. These compounds exist in many tautomeric and enantiomeric forms. The R and S enantiomeric position ("chiral center") exists at the point of attachment of the sulfonate group. The tautomeric forms include, for example:

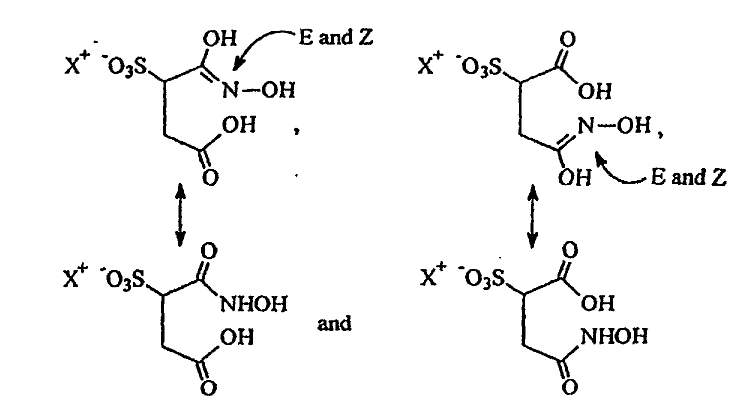

Enolization may also occur such as:

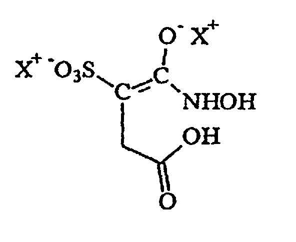

All of these structural variants in the structure of the sulfo-N-hydroxamic acid precursor compounds are of course expected to be statistically or potentially present as part of any composition containing the primary sulfo-NHS compound of interest, the presence of these variants being at least partially dependent upon the environment, pH conditions or other system influences on the composition.

At least three other distinguishing aspects of the present invention also appear to be present in the compounds formed using the process of the present invention. These aspects may be individually or jointly present in the compositions formed using the process of the invention. As previously noted, the process of the prior art used to produce sulfo-NHS produced the imide analog of the sulfo-NHS compound as a by-product, that succinimide (hydrogen analog) compound being represented by the formula: 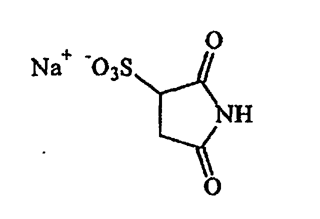 It is typically present in amounts greater than 1.0% by weight of the sulfo-NHS compound, even after repeated purification and the attempt to produce a pure sulfo-NHS composition in the process of the prior art. Even commercial sulfo-NHS contains this impurity, and of the 2-5% impurity in the commercial sulfo-NHS composition, this succinimide compound may be the largest contaminant. The process of the present invention produces a route to the sulfo-NHS compound and class of compounds which does not produce the succinimide contaminant. Therefore sulfo-NHS compositions which do not contain the succinimide counterpart are previously unknown and, to date, have been produced only by means of the process described in the present invention. The sulfo-NHS compounds produced using the process of the present invention may therefore be characterised as novel by the presence of less than 1% by weight of the succinimide counterpart, preferably less than 0.5% or less than 0.25%, and most preferably less than 0.1% down to 0% by weight of the succinimide.

Secondly, the resulting product from the process of the prior art which produces sulfo-NHS and the commercially available sulfo-NHS materials are amorphous solids. This is thought to be a result of the particular precipitation step used in the final step of the prior art process. The continuous batch process of the present invention produces distinct, white crystals. The clearly crystalline form of the sulfo-NHS compounds produced by this process is also distinct from the amorphous solid sulfo-NHS compounds produced by the prior art process.

Additionally, the sulfo-NHS compounds produced using the process of the present invention may be separated into the separate enantiomers by a chiral separator. The R and S enantiomers may be separated into more highly purified isomers. This can be very important, as the R and S enantiomers will normally be used to make conjugates for use in assays, e.g., blotting or Elisa based immunoassays, etc. An unpurified mixture of enantiomers may provide a material with only 50% activity, especially if only one of the R and S enantiomers might be reactive towards a site. By providing a sulfo-NHS composition which may selectively contain higher proportions of R or S enantiomers, up to nearly 100% of either of the enantiomers, the resultant conjugates may be created to form enhanced functional systems. This can enable systems which are tailored for their degree of activity, without having to alter the remaining portions of the composition. For example, if an assay system were provided with a nominal-activity of 10, using a 50/50 mixture of R and S enantiomers, the activity might be adjusted from 0 to 20 by appropriate selection of concentrations of the R or S enantiomer which was active in a particular assay. As previously mentioned, this chiral separation may be performed in a conventional manner (such as HPLC chromatography on a chiral column).

### EXAMPLES

The following examples show the synthesis and then the use of the sulfosuccinic acid and sulfosuccinimide compounds formed using the process of the present invention in the manufacture of chemically active species with known commercial utility. Many of the prepared compounds are commercially available by other synthetic routes, but would retain many of the impurities of the prior art synthetic procedures, such as the hydrogen counterpart of the N-hydroxy-succinimide discussed above.

### Example 1

### Preparation of Tetrahydro-2,5-dioxo-3-furansulfonic acid sodium salt

Tetrahydro-2,5-dioxo-3-furansulfonic acid sodium salt was prepared by stirring 1.71 moles of sulfosuccinic acid with 1.87 moles of sodium hydroxide at 50°C until a milky suspension formed and then adding 1 kg acetic anhydride over 6 hours. After cooling, 1 L of ethyl acetate was added and the mixture was filtered. The filter cake was washed with 500 mL ethyl acetate. On drying 344.32 g of white crystalline product was obtained. ¹³C-NMR (d6-DMSO) 171.3, 167.2, 60.4, 34.0 ppm.

### Example 2

### Preparation of Sulfosuccinic monohydroxamic acid sodium salt

Sulfosuccinic monohydroxamic acid sodium salt was prepared by reacting tetrahydro-2,5-dioxo-3-furansulfonic acid sodium salt with hydroxylamine. 38.2 g hydroxylamine hydrochloride and 35.5 g potassium hydroxide were stirred at 5°C in 300 mL methanol. A white precipitate formed and was filtered off.

The above methanol solution of hydroxylamine was slowly added to 90 g tetrahydro-2,5-dioxo-3-furansulfonic acid sodium salt in 100 mL methanol at 5°C. The mixture was stirred overnight and stripped to give 111.77 g white solid product. ¹H-NMR (D₂O) 3.80-4.08 ppm m, 2.63-2.94 ppm m.

### Example 3

### Preparation of Sulfo-N-hydroxysuccinimide sodium salt

A suspension of 237.3 g sulfosuccinic monohydroxamic acid sodium salt in 700 g glacial acetic acid was slowly heated. At 70°C TLC(thin layer chromatography, silica gel developed with 10% water/acetone) showed sulfo-N-hydroxysuccinimide sodium salt forming. A precipitate of sulfo-N-hydroxysuccinimide sodium salt formed at 80°C after 2 hours which was cooled to room temperature and isolated by filtration to give 68 g sulfo-N-hydroxysuccinimide as a white solid. The filtrate was diluted with 1 L of acetone and the mixture was filtered again to recover an additional 70 g of sulfo-N-hydroxysuccinimide.

The combined product was purified by crystallization from 20% aqueous acetic acid. The product was dried under vacuum at 40-55°C. (98.4% by quantitative ¹H-NMR in D₂O with maleic acid internal standard). The product contained less than 0.1% sulfo-succinimide impurity by C-18 ion pair HPLC.

### Example 4

### Preparation of 1-Hydroxy-2,5-dioxo-3-pyrrolidinesulfonic acid

1-Hydroxy-2,5-dioxo-3-pyrrolidinesulfonic acid was prepared as follows. 17 g of the sodium salt of sulfo-N-hydroxysuccinimide was dissolved in 50 mL water and applied to a preformed 50 g column of Aglx8(OH-) anion exchange resin. The column was rinsed with 760 mL deionized water and the 1-hydroxy-2,5-dioxo-3-pyrrolidinesulfonic acid was eluted off with 200 mL 25% hydrochloric acid monitoring fractions by UV at 210 nm. The sulfo-N-hydroxysuccinimide containing fractions were stripped under high vacuum to produce 2.11 g viscous oil.

### Example 5

### Dicyclohexylcarbodiimide ring closure(dehydration) of Sulfosuccinic monohydroxamic acid sodium salt

2 g of sulfosuccinic monohydroxamic acid sodium salt in 150 mL dimethylformamide (DMF) was stirred with 1.75 g dicyclohexylcarbodiimide at 25°C for 18 hours. The mixture turned an orange shade. The dicyclohexylurea precipitate was filtered off and the DMF solution was stripped under high vacuum. Ion pair HPLC showed sulfo-N-hydroxysuccinimide had formed.

### Example 6

### N,N-Carbonyldiimidazole ring closure of Sulfosuccinic monohydroxamic acid sodium salt

1 g of sulfosuccinic monohydroxamic acid sodium salt in 50 mL dimethylsulfoxide (DMSO) was stirred with 0.68 g N,N-carbonyldiimidazole. Carbon dioxide gas evolved over 30 minutes. The DMSO solution was stripped under high vacuum and 20 mL isopropanol was added. The white precipitate was filtered off and dried to give 0.3 g sulfo-N-hydroxysuccinimide as shown by ¹H-NMR.

### Example 7

### Reaction of Sulfosuccinic monohydroxamic acid sodium salt with acetic anhydride

Sulfosuccinic monohydroxamic acid sodium salt prepared from 300 g tetrahydro-2,5-dioxo-3-furansulfonic acid sodium salt (as in Example 2) was treated with 650 mL acetic anhydride for 4 hours at 55°C while distilling off water and acetic acid under vacuum. 1.5 L ethyl acetate was added and the white powder was filtered off and dried under a vacuum at 50°C to give 305.53 g product. The product was mostly sulfo-N-acetoxy succinimide sodium salt by TLC comparison with an authentic standard and by NMR.

### Example 8

### Preparation of Sulfo-succinimide sodium salt

1.24 g maleimide, 1.22 g sodium metabisulfite and 30 mL water were stirred 30 min. UV spectra in water showed the formation of a peak at 197 nm. The solution was stripped to give 1.99 g white crystals. Ion pair HPLC gives a single peak at 5.57 min. (sulfo-N-hydroxysuccinimide 6.17 min.)

### Example 9

### Preparation of Sulfo-N-hydroxysuccinimide sodium salt via hydrolysis of sulfo-N-acetoxy succinimide sodium salt

30 g Sulfo-N-acetoxy succinimide sodium salt was dissolved in 200 mL acetic acid and 0.8 mL water. The mixture was heated to reflux for 30 min., cooled and 200 mL ethyl acetate was added. The white crystals were filtered off and dried under a vacuum to give 26.45 g product which was mostly sulfo-N-hydroxysuccinimide by ¹H-NMR.

### Example 10

### Preparation of 3-methyl-2-sulfosuccinic acid sodium salt (cis and trans diastereomers)

3-Methyl-2-sulfosuccinic acid sodium salt was prepared as follows. 20g Methylsuccinic anhydride was dissolved in 40g 1,2-dichloroethane. 15.4g solid sulfur trioxide was melted and added in portions to 140g 1,2-dichloroethane in an addition funnel. The sulfur trioxide solution was added slowly to the anhydride solution and stirred overnight. 50mL water was then added, followed by 7g sodium hydroxide dissolved in 50 mL water. The layers were separated and the 1,2-dichloroethane layer was again extracted with 50 mL water. The combined water layers were stripped to give 42.2g yellowish solid. ¹H-NMR (D₂O) 2.65-2.80 ppm m, 2.47-2.58 ppm m, 1.04 ppm d J=7.07 Hz.

### Example 11

### Preparation of Tetrahvdro-2.5-dioxo-4-methyl-3-furansulfonic acid sodium salt (cis and trans diastereomers)

40g 3-methyl-2-sulfosuccinic acid sodium salt (cis and trans diastereomers) was stirred with 50g acetic anhydride and then heated to 86 degrees Centigrade while distilling over acetic acid for 1.5 hours. 100 mL ethyl acetate was added and the mixture was filtered. The precipitate was washed with 100 mL ethyl acetate. The precipitate was dried to produce 16.0g yellowish-white solid. ¹H-NMR (d6-DMSO) 3.40 ppm d J=10.52 Hz, 2.92-2.98 ppm m, 1.28-1.31 ppm m J=7.12 Hz.

### Example 12

### Preparation of 3-methyl-2-sulfo-N-hydroxysuccinimide sodium salt (cis and trans diastereomers)

To 2.86g potassium hydroxide in 50 mL of methanol cooled to 5 degrees Centigrade was added 3.53g hydroxylamine hydrochloride. A white precipitate was filtered off.

To 10g of tetrahydro-2,5-dioxo-4-methyl-3-furansulfonic acid sodium salt (cis and trans diastereomers) and 50 mL methanol at 5 degrees Centigrade was added the above hyroxylamine solution. The mixture was stirred overnight, allowing the mixture to warm to room temperature. The suspension was stripped to give 10.97g product. TLC (Thin layer chromotography) using 10% water/acetone on silica gel gives a 0.05 r.f. spot reacting with potassium permanganate (turning yellowish white). The crude product was recrystallized from 100 mL warm acetic acid to give 9.27g white crystals after washing with 150 mL ethyl acetate. ¹H-NMR (D2O)-4.02 ppm d, 3.88 ppm d, 3.00-3.14 ppm m, 1.35 ppm (cis) d J=7.5 Hz, 1.19 ppm (trans) d J=7.0 Hz. The ratio of trans to cis diastereomers was 60:40 by proton NMR integration.

The derivatives of the sulfo-N-Hydroxysuccinimides used in the art, when made from the sulfosuccinimide-free analogs (free of the hydrogen analogs) are distinguishable from the otherwise chemically identical derivatives by the absence of the analogs. The esterification derivatives, e.g.,

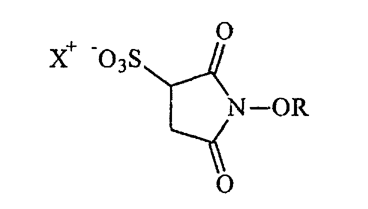

Wherein X⁺ is a cation and R is H or an organic group, or especially any organic group formed from a compound R-OH wherein R-OH is an acid, and the symbolic extraction of the hydroxyl forms an ester group with the residue (less - OR) of the central nucleus.

Examples of preferred R-OH compounds are acetic acid, LC-biotin suberic acid, biotin, suberic acid, 4-[N-maleimidomethyl]-cyclohexane-1-carboxylic acid, and the like, are also free of the hydrogen analog (the sulfosuccinimide as opposed to the sulfo-N-hydroxy succinimide) while the prior art ester derivatives would have some of that contaminant present. Therefore, the ester derivatives which are free of the sulfosuccinimide analog are also novel compositions.

### Example 13

### Preparation of SNHS

Sulfosuccinic anhydride sodium salt (2.56 g) and hydroxylamine hydrochloride (2.13 g) are heated in DMAC (30 ml) to 80 C. TLC after 30 min shows formation of the known UV active sulfo-NHS. TLC system: 35-40% methanol in acetone or 5% water in methanol or 6% water in acetone; silica gel plates. The product was not isolated from this reaction.

### Example 14

### Preparation of SNHS

The hydroxamic acid (237 g) in glacial acetic acid (700 g) is heated to 80 C for 2 hours under nitrogen. The reaction mixture is cooled to room temperature and filtered to give 68 g of SNHS as a white solid. The filtrate is diluted with 1 liter of acetone and product is purified by crystallization from 20% water in acetic acid.

### Example 15

### Preparation of SNHS via Diester

Sulfosuccinic acid (70% in water, 211 g, 0.75 mol) and methanol (256 g, 10.7 eq) are combined in a 1 liter flask; the reaction exotherms as the formation of methyl ester starts. The solution is stirred overnight at room temperature. Hydroxylamine hydrochloride (54.7 g, 0.79 mol, 1.05 eq) is added followed by sodium hydroxide pellets (34 g). The reaction exotherms to 40 C upon addition of the base. TLC analysis (6% water in acetone, silica gel plate) after stirring 3 days indicates formation of the UV active sulfo-NHS. TLC after 4 weeks clearly shows the UV active sulfo-NHS along with permanganate active hydroxamic acid/ester. TLC Rf values after two elutions: S-NHS = 0.6-0.7 streak, UV active; hydroxamic acid/ester: Rf = 0.2, non-UV active, potassium permanganate active.

### Example 16

### Preparation of SNHS via Diester

Sulfosuccinic acid (70% in water, 56 g, 0.2 mol) and methanol (100 ml, 12.3 eq) are combined in a 250 ml flask; the reaction exotherms as the formation of methyl ester starts. The solution is refluxed for two hours and then evaporated on a rotary evaporator to remove water and methanol. The product ester is a clear colorless liquid of moderate viscosity.

The product is treated with 160 g of a 5.5 wt% solution of hydroxlamine in methanol. The reaction exotherms immediately as the hydroxylamine is added. TLC analysis (6% water in acetone, silica gel plate) after stirring overnight indicates formation of the UV active sulfo-NHS. TLC after 3 days clearly shows the UV active sulfo-NHS. TLC Rf values: SNHS = 0.6-0.7 streak, UV active.

Another advantage can be readily appreciated in the practice of the present invention as compared to the use of the sulfo-N-hydroxysuccinimide materials of the prior art. Because the materials of the prior art contained the sulfo-N-succinimide impurity in uncontrolled and varying amounts, critical qaulitative and/or critical quantitative comparisons could never be made using different lots of sulfo-N-hydroxysuccinimide. The results, because of the variation in concentration of the sulfo-N-succinimide, would vary by detectible but not predictable amounts. Because of the uniformity of lot-to-lot batches of sulfo-N-hydroxysuccinimide and the lack of sulfo-N-succinimide impurity, the sulfo-N-hydroxysuccinimide materials produced using the process of the present invention can be used in critical qualitative and critical quantitative assays using materials from different lots. This adds a new use to the sulfo-N-hydroxysuccinimide compounds.

## Claims

1. A synthetic process comprising the steps of:
a) cyclizing a sulfo-succinic acid compound to form a monocyclic first product, and
b) opening a ring of said monocyclic first product,
**characterised by** opening the ring of said monocyclic first product in the presence of a hydroxylamine to form a sulfo-hydroxamic acid

2. A synthetic process of claim 1 comprising the steps of:
a) cyclizing a sulfo-succinic acid having the central nucleus of: 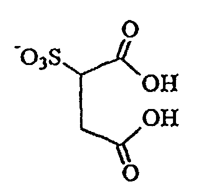 to form a monocyclic first product,
b) opening a ring of said monocyclic first product in the presence of hydroxylamine to form a sulfo-hydroxamic acid.

3. A synthetic process for synthesizing a monocyclic sulfo-N-hydroxysuccinimide comprising a process **characterised by** comprising combinations of steps selected from the group consisting of:
A -
a) esterifying a sulfosuccinic acid to form a diester first product, and
b) reacting said diester first product with hydroxylamine to form a sulfo-N-hydroxy succinimide, and
B -
a) cyclizing a sulfo-succinic acid to form a monocyclic first product,
b) opening a ring of said monocyclic first product in the presence of a hydroxylamine to form a sulfo-hydroxamic acid,
c) dehydrating said sulfo-hydroxamic acid to form a monocyclic sulfo-N-hydroxy-succinimide.

4. The process of claim 3 wherein said sulfo-hydroxamic acid comprises a compound of the general formula: 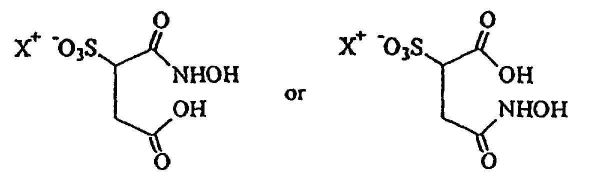 wherein X⁺ is a cation.

5. The process of claim 1 wherein said sulfo-hydroxamic acid is then dehydrated to form a monocyclic sulfo-N-hydroxy-succinimide.

6. The process of claim 5 wherein said sulfo-N-hydroxy succinimide comprises the central nucleus of: 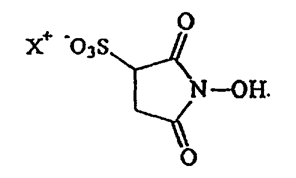 wherein X⁺ is a cation.

7. The process of claim 5 or 6 wherein said dehydration of the sulfohydroxamic acid occurs in the presence of methanol, water, acetic acid, acetic anhydride, dicyclohexylcarbodiimide and/or carbonyldiimidazole.

8. The process of claim 1 wherein said opening a ring of said monocyclic first product in the presence of hydroxylamine acid complex to form a sulfo-hydroxamic acid is done in the presence of excess alcohol to produce a sulfo-hydroxamic acid ester as a partial product.

9. A sulfo-hydroxamic acid having the general formula: 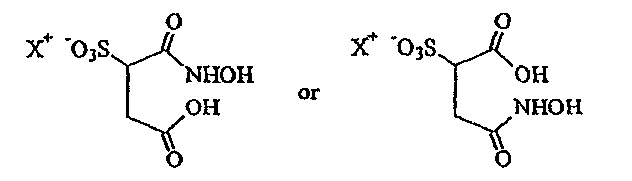 wherein X⁺ is a cation.

10. The process of claim 2 wherein said hydroxamic acid is within the same Step cyclized and csterified.

11. A process comprising hydrolyzing the succinimide having the general formula: 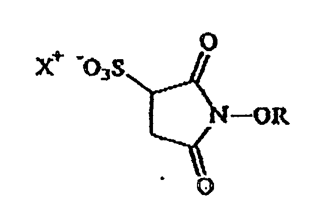 wherein X⁺ is a cation and R is an organic group formed from a compound R-OH wherein R-OH is an acid, wherein said succinimide is present as a white crystalline powder.

12. A synthetic process according to claim 3 comprising the steps of:
a) esterifying a sulfosuccinic acid to form a diester first product, and
b) reacting said diester first product with hydroxylamine to form a sulfo-N-hydroxy succinimide.

13. A synthetic process of claim 12 wherein esterifying a sulfosuccinimide to form a diester first product comprises:
a) esterifying a sulfosuccinic acid having the central nucleus of: 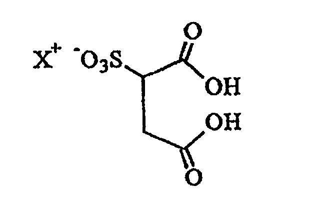 to form a diester first product, wherein X⁺ is a cation.

14. The process of claim 12 wherein said hydroxylamine is added to said diester first product.

15. The process of claim 12 wherein said hydroxylamine is formed in the presence of said diester first product.

16. The process of claim12 wherein a sulfo-hydroxamic acid is formed in a reaction of said diester first product with hydroxylamine to form a sulfo-N-hydroxy succinimide.

17. The process of claim 16 wherein said sulfo-hydroxamic acid comprises a compound of the general formula: 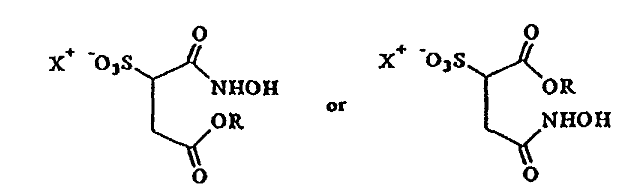 wherein X⁺ is a cation, and R is hydrogen or alkyl group.

18. The process of claim 16 wherein said sulfo-hydroxamic acid is cyclized to form a monocyclic sulfo-N-hydroxysuccinimide.

19. The process of claim 12 wherein said sulfo-N-hydroxysuccinimide comprises the central nucleus of: 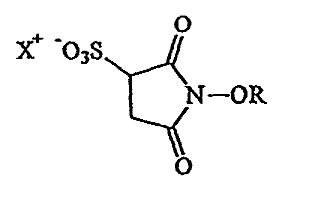 wherein R-OH is an acid, and the OH (hydroxyl) which, when
extracted leaves the group R- to form an ester in the compound and X⁺ is a cation.

20. The process of claim 18 wherein said dehydration of the sulfohydroxamic acid occurs in the presence of methanol, water, acetic acid, dicyclohexylcarbodiimide and/or carbonyldiimidazole.

21. The process of claim 12, wherein said sulfo-N-hydroxysuccinimide is produced in the absence of a sulfosuccinimide of the formula: 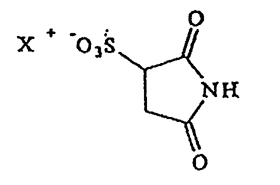 wherein X⁺ is a cation.

22. The process of claim 12 wherein said diester first product comprises a compound having the formula: 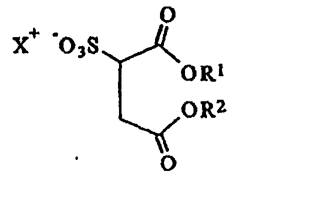 wherein X⁺ is a cation, and R¹ and R² are independently selected from the group consisting of alkyl and aryl groups.

23. The process of claims 13 or 16 wherein said diester first product comprises a compound having the formula: 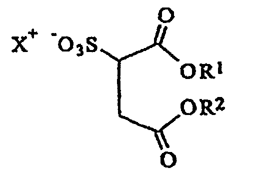 wherein X⁺ is a cation, and R¹ and R² are independently selected from the group consisting of alkyl and phenyl groups.

24. The method of claims 12 or 13 wherein the sulfo-N-hydroxysuccinimide is present in the absence of sulfo-succinimide.

25. The method of claims 12, 13 or 15 wherein the sulfo-N-hydroxysuccinimide has proportions of R and S enantiomers hydroxysuccinimide which are present as 100 to 50% R or S enantiomers.

26. A process for synthesizing sulfo-N-hydroxysuccinimides, said process **characterised by** comprising the step of:
a) reacting a sulfosuccinate diester with hydroxylamine to form a sulfo-N-hydroxysuccinimide.

27. The process of claim 12 wherein said hydroxylamine comprises hydroxylamine hydrochloride, added to said sulfosuccinate diester or formed *in situ*.

28. A synthetic process comprising the steps of:
a) esterifying a sulfo-succinimide compound to form a diester first product, and
b) further **characterised by** cyclizing said diester first product to form a sulfo-N-hydroxysuccinimide.

29. A synthetic process comprising the step of cyclizing a diester of sulfosuccinic acid to form a sulfo-N-hydroxy succinimide.

30. The process of claim 29 wherein said cyclizing is done in the presence of hydroxylamine.

31. The process of claim 30 wherein said hydroxylamine is hydroxylamine hydrochloride.

## Patentansprüche

1. Syntheseverfahren, welches die Schritte
a) Cyclisierung einer Sulfobernsteinsäureverbindung zur Bildung eines monocyclischen ersten Produktes,
b) Öffnen eines Ringes des monocyclischen ersten Produkts
umfasst, **gekennzeichnet durch** Öffnen des Ringes des monocyclischen ersten Produktes in Gegenwart eines Hydroxylamins zur Bildung einer Sulfohydroxamsäure.

2. Syntheseverfahren nach Anspruch 1, welches die Schritte
a) Cyclisierung einer Sulfobernsteinsäure mit dem zentralen Kern 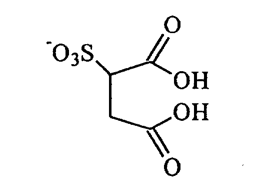 um ein monocyclisches erstes Produkt zu bilden,
b) Öffnen eines Rings des ersten monocyclischen Produktes in Gegenwart von Hydroxylamin zur Bildung einer Sulfohydroxamsäure
umfasst.

3. Syntheseverfahren zur Synthese eines monocyclischen Sulfo-N-hydroxysuccinimids, welches ein Verfahren umfasst, das durch die Kombination einer Auswahl der folgenden Schritte gekennzeichnet ist:
A-
a) Veresterung von Sulfobernsteinsäure zur Bildung eines ersten Diesterprodukts, und
b) Reaktion des ersten Diesterprodukts mit Hydroxylamin zur Bildung eines Sulfo-N-hydroxysuccinimids, und
B-
a) Cyclisierung einer Sulfobernsteinsäure zur Bildung eines monocyclischen ersten Produkts,
b) Öffnen eines Ringes des monocyclischen ersten Produktes in Gegenwart eines Hydroxylamins zur Bildung einer Sulfohydroxamsäure,
c) Wasserabspaltung aus der Sulfohydroxamsäure zur Bildung eines monocyclischen Sulfo-N-hydroxysuccinimids.

4. Verfahren nach Anspruch 3, worin die Sulfohydroxamsäure eine Verbindung der allgemeinen Formel 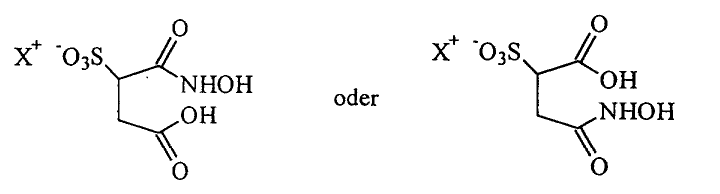 umfasst, in der X⁺ ein Kation ist.

5. Verfahren nach Anspruch 1, in welchem aus der genannten Sulfohydroxamsäure dann Wasser abgespalten wird, um ein monocyclisches Sulfo-N-hydroxysuccinimid zu bilden.

6. Verfahren nach Anspruch 5, worin das Sulfo-N-hydroxysuccinimid den zentralen Kern 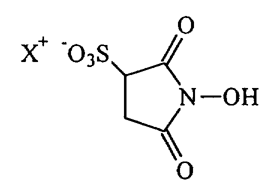 umfasst, in dem X⁺ ein Kation ist.

7. Verfahren nach Anspruch 5 oder 6, worin die Wasserabspaltung aus der Sulfohydroxamsäure in Gegenwart von Methanol, Wasser, Essigsäure, Essigsäureanhydrid, Dicyclohexylcarbodiimid und/oder Carbonyldiimidazol erfolgt.

8. Verfahren nach Anspruch 1, worin die Ringöffnung des monocyclischen ersten Produkts in Gegenwart eines Hydroxylaminsäurekomplexes zur Bildung einer Sulfohydroxamsäure in Gegenwart eines Alkoholüberschusses durchgeführt wird, um einen Sulfohydroxamsäureester als Teilprodukt zu bilden.

9. Sulfohydroxamsäure der allgemeinen Formel 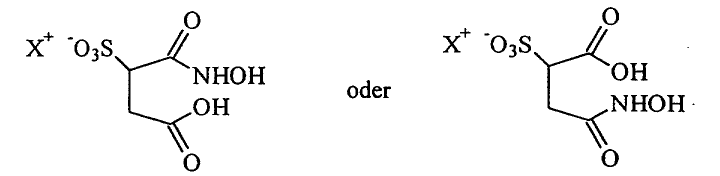 worin X⁺ ein Kation ist.

10. Verfahren nach Anspruch 2, worin die Hydroxamsäure im gleichen Schritt cyclisiert und verestert wird.

11. Verfahren, welches die Hydrolyse eines Succinimids der allgemeinen Formel 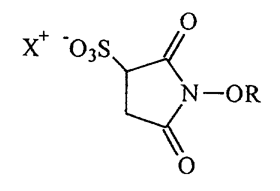 umfasst, worin X⁺ ein Kation und R ein aus einer Säure der Formel R-OH gebildeter organischer Rest ist und in welchem das Succinimid als weißes kristallines Pulver vorliegt.

12. Syntheseverfahren nach Anspruch 3, welches die Schritte
a) Veresterung einer Sulfobernsteinsäure zur Bildung eines ersten Diesterprodukts, und
b) Reaktion des ersten Diesterprodukts mit Hydroxylamin zur Bildung eines Sulfo-N-hydroxysuccinimids
umfasst.

13. Syntheseverfahren nach Anspruch 12, worin die Veresterung von Sulfosuccinimid zur Bildung eines ersten Diesterprodukts
a) die Veresterung einer Sulfobernsteinsäure mit dem zentralen Kern 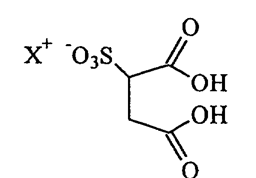 zur Bildung eines ersten Diesterprodukts umfasst, worin X⁺ ein Kation ist.

14. Verfahren nach Anspruch 12, worin Hydroxylamin zum ersten Diesterprodukt gegeben wird.

15. Verfahren nach Anspruch 12, worin Hydroxylamin in Gegenwart des ersten Diesterprodukts gebildet wird.

16. Verfahren nach Anspruch 12, worin Sulfohydroxamsäure in einer Reaktion des ersten Diesterprodukts mit Hydroxylamin zur Bildung von Sulfo-N-hydroxysuccinimid gebildet wird.

17. Verfahren nach Anspruch 16, worin die Sulfohydroxamsäure eine Verbindung der allgemeinen Formel 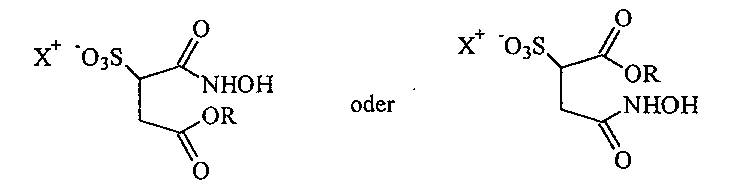 umfasst, in der X⁺ ein Kation und R Wasserstoff oder ein Alkylrest ist.

18. Verfahren nach Anspruch 16, worin die Sulfohydroxamsäure zur Bildung eines monocyclischen Sulfo-N-hydroxysuccinimids cyclisiert wird.

19. Verfahren nach Anspruch 12, worin das Sulfo-N-hydroxysuccinimid den zentralen Kern 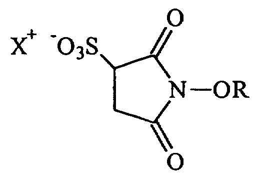 umfasst, in welchem R-OH eine Säure ist, und das OH (Hydroxyl) bei Abspaltung die Gruppe R- verlässt, um einen Ester in der Verbindung bilden, und X⁺ ein Kation ist.

20. Verfahren nach Anspruch 18, worin die Wasserabspaltung aus der Sulfohydroxamsäure in Gegenwart von Methanol, Wasser, Essigsäure, Dicyclohexylcarbodiimid und/oder Carbonyldiimidazol erfolgt.

21. Verfahren nach Anspruch 12, worin das Sulfo-N-hydroxysuccinimid in Abwesenheit eines Sulfosuccinimids der Formel 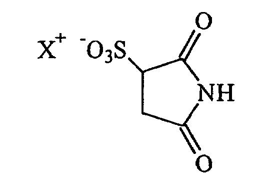 hergestellt wird, worin X⁺ ein Kation ist.

22. Verfahren nach Anspruch 12, worin das erste Diesterprodukt eine Verbindung der Formel 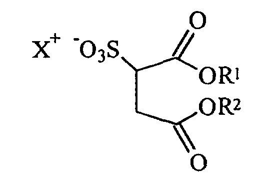 umfasst, in welcher X⁺ ein Kation ist, und R¹ und R² unabhängig voneinander aus der Gruppe bestehend aus Alkyl- und Arylresten gewählt sind.

23. Verfahren nach Anspruch 13 oder 16, in welchem das genannte erste Diesterprodukt eine Verbindung der Formel 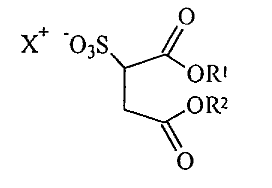 umfasst, in der X⁺ ein Kation ist und R¹ und R² voneinander unabhängig aus der Gruppe bestehend aus Alkyl- und Phenylresten gewählt sind.

24. Verfahren nach Anspruch 12 oder 13, in welchem Sulfo-N-hydroxysuccinimid in Abwesenheit von Sulfosuccinimid vorliegt.

25. Verfahren nach Anspruch 12, 13 oder 15, in welchem das Sulfo-N-hydroxysuccinimid Anteile von Rund S-Enantiomeren des Hydroxysuccinimids enthält, welche als 100% bis 50% R- oder S-Enantiomere vorliegen.

26. Verfahren zur Synthese von Sulfohydroxysuccinimiden, **dadurch gekennzeichnet, dass** es den Schritt
a) Reaktion eines Sulfobernsteinsäurediesters mit Hydroxylamin zur Synthese eines Sulfo-N-hydroxysuccinimids
umfasst.

27. Verfahren nach Anspruch 12, worin das Hydroxylamin Hydroxylaminhydrochlorid umfasst, welches dem Sulfobernsteinsäurediester zugesetzt wird oder *in situ* gebildet wird.

28. Syntheseverfahren, welches die Schritte
a) Veresterung einer Sulfosuccinimidverbindung zur Bildung eines ersten Diesterproduktes umfasst und
b) weiterhin durch Cyclisierung des ersten Diesterproduktes zur Bildung von Sulfo-N-hydroxysuccinimid gekennzeichnet ist.

29. Syntheseverfahren, welches den Schritt der Cyclisierung eines Diesters der Suifobernsteinsäure zur Bildung eines Sulfo-N-hydroxysuccinimids umfasst.

30. Verfahren nach Anspruch 29, worin die Cyclisierung in Gegenwart von Hydroxylamin durchgeführt wird.

31. Verfahren nach Anspruch 30, worin das Hydroxylamin Hydroxylaminhydrochlorid ist.

## Revendications

1. Procédé de synthèse, qui comprend les étapes de :
a) cyclisation d'un composé d'acide sulfosuccinique pour former un premier produit monocyclique, et
b) ouverture d'un cycle dudit premier produit monocyclique,
**caractérisé par** l'ouverture du cycle dudit premier produit monocyclique en présence d'une hydroxylamine pour former un acide sulfo-hydroxamique.

2. Procédé de synthèse suivant la revendication 1, qui comprend les étapes de :
a) cyclisation d'un acide sulfo-succinique ayant le noyau central de : 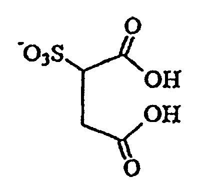 pour former un premier produit monocyclique,
b) ouverture d'un cycle dudit premier produit monocyclique en présence d'hydroxylamine pour former un acide sulfo-hydroxamique.

3. Procédé de synthèse pour la synthèse d'un sulfo-N-hydroxysuccinimide monocyclique comprenant un procédé **caractérisé en ce qu'**il comprend des combinaisons d'étapes choisies dans le groupe consistant en :
A-
a) estérification d'un acide sulfosuccinique pour former un premier produit diester, et
b) réaction dudit premier produit diester avec une hydroxylamine pour former un sulfo-N-hydroxy succinimide, et
B-
a) cyclisation d'un acide sulfo-succinique pour former un premier produit monocyclique,
b) ouverture d'un cycle dudit premier produit monocyclique en présence d'une hydroxylamine pour former un acide sulfo-hydroxamique,
c) déshydratation dudit acide sulfo-hydroxamique pour former un sulfo-N-hydroxy-succinimide monocyclique.

4. Procédé suivant la revendication 3, dans lequel ledit acide sulfohydroxamique comprend un composé de formule générale : 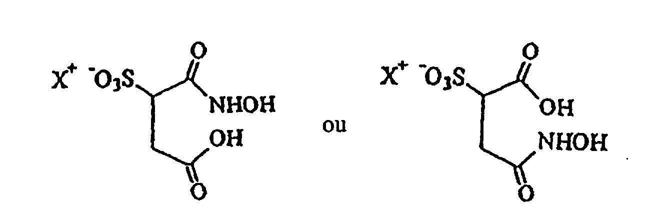 dans laquelle X⁺ est un cation.

5. Procédé suivant la revendication 1, dans lequel ledit acide sulfohydroxamique est ensuite déshydraté pour former un sulfo-N-hydroxysuccinimide monocyclique.

6. Procédé suivant la revendication 5, dans lequel ledit sulfo-N-hydroxysuccinimide comprend le noyau central de : 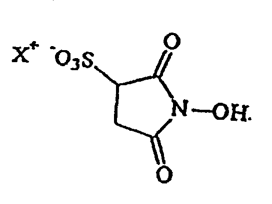 dans lequel X⁺ est un cation.

7. Procédé suivant la revendication 5 ou 6, dans lequel ladite déshydratation de l'acide sulfohydroxamique a lieu en présence de méthanol, d'eau, d'acide acétique, d'anhydride acétique, de dicyclohexylcarbodiimide et/ou de carbonyldiimidazole.

8. Procédé suivant la revendication 1, dans lequel ladite ouverture d'un cycle dudit premier produit monocyclique en présence d'un complexe acide d'hydroxylamine pour former un acide sulfo-hydroxamique est effectuée en présence d'un excès d'alcool pour produire un ester d'acide sulfo-hydroxamique en tant que produit partiel.

9. Acide sulfo-hydroxamique ayant la formule générale : 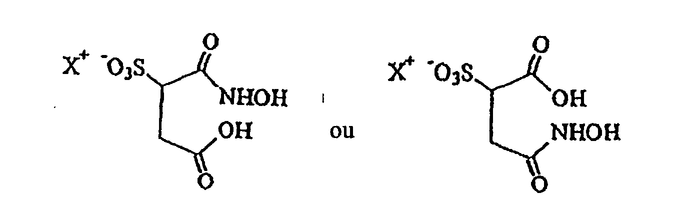 dans laquelle X⁺ est un cation.

10. Procédé suivant la revendication 2, dans lequel ledit acide hydroxamique est soumis à une cyclisation et à une estérification dans la même étape.

11. Procédé comprenant l'hydrolyse du succinimide ayant la formule générale : 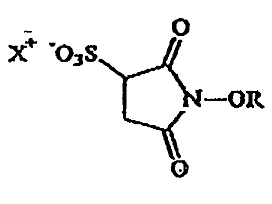 dans laquelle X⁺ est un cation et R est un groupe organique formé à partir d'un composé R-OH dans lequel R-OH est un acide, dans lequel ledit succinimide est présent sous la forme d'une poudre cristalline blanche.

12. Procédé de synthèse suivant la revendication 3, qui comprend les étapes de :
a) estérification d'un acide sulfosuccinique pour former un premier produit diester, et
b) réaction dudit premier produit diester avec de l'hydroxylamine pour former un sulfo-N-hydroxy succinimide.

13. Procédé de synthèse suivant la revendication 12, dans lequel l'estérification d'un sulfosuccinimide pour former un premier produit diester comprend :
a) l'estérification d'un acide sulfosuccinique ayant le noyau central de : 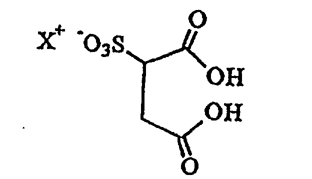 pour former un premier produit diester, dans lequel X⁺ est un cation.

14. Procédé suivant la revendication 12, dans lequel ladite hydroxylamine est ajoutée audit premier produit diester.

15. Procédé suivant la revendication 12, dans lequel ladite hydroxylamine est formée en présence dudit premier produit diester.

16. Procédé suivant la revendication 12, dans lequel un acide sulfohydroxamique est formé dans une réaction dudit premier produit diester avec une hydroxylamine pour former un sulfo-N-hydroxy succinimide.

17. Procédé suivant la revendication 16, dans lequel ledit acide sulfo hydroxamique comprend un composé de formule générale : 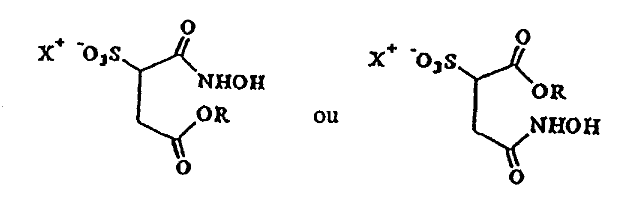 dans laquelle X⁺ est un cation, et R est un atome d'hydrogène ou un groupe alkyle.

18. Procédé suivant la revendication 16, dans lequel ledit acide sulfo hydroxamique est soumis à une cyclisation pour former un sulfo-N-hydroxysuccinimide monocyclique.

19. Procédé suivant la revendication 12, dans lequel ledit sulfo-N-hydroxysuccinimide comprend le noyau central de : 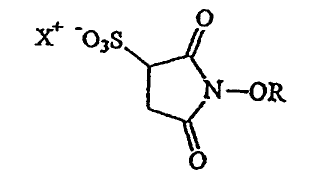 dans lequel R-OH est un acide, et le groupe OH (hydroxy) qui, lorsqu'il est extrait, quitte le groupe R- pour former un ester dans le composé, et X⁺ est un cation.

20. Procédé suivant la revendication 18, dans lequel ladite déshydratation de l'acide sulfohydroxamique a lieu en présence de méthanol, d'eau, d'acide acétique, de dicyclohexylcarbodiimide et/ou de carbonyldiimidazole.

21. Procédé suivant la revendication 12, dans lequel ledit sulfo-N-hydroxysuccinimide est produit en l'absence d'un sulfosuccinimide de formule : 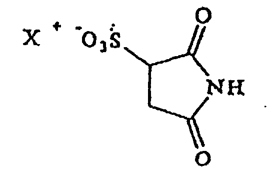 dans laquelle X⁺ est un cation.

22. Procédé suivant la revendication 12, dans lequel ledit premier produit diester comprend un composé ayant la formule : 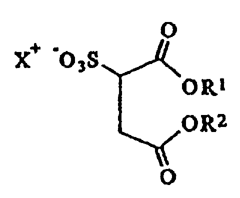 dans laquelle X⁺ est un cation, et R¹ et R² sont choisis indépendamment dans le groupe consistant en groupes alkyle et aryle.

23. Procédé suivant la revendication 13 ou 16, dans lequel ledit premier produit diester comprend un composé ayant la formule : 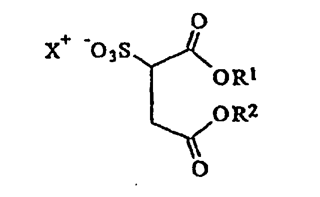 dans laquelle X⁺ est un cation, et R¹ et R² sont choisis indépendamment dans le groupe consistant en groupes alkyle et phényle.

24. Procédé suivant les revendications 12 ou 13, dans lequel le sulfo-N-hydroxysuccinimide est présent en l'absence de sulfo-succinimide.

25. Procédé suivant les revendications 12, 13 ou 15, dans lequel ledit sulfo-N-hydroxysuccinimide a des proportions d'énantiomères R et S d'hydroxysuccinimide présents sous la forme de 100 à 50% d'énantiomères R ou S.

26. Procédé pour la synthèse de sulfo-N-hydroxysuccinimides, ledit procédé étant **caractérisé en ce qu'**il comprend l'étape de :
a) réaction d'un diester sulfosuccinate avec une hydroxylamine pour former un sulfo-N-hydroxysuccinimide.

27. Procédé suivant la revendication 12, dans lequel ladite hydroxylamine comprend le chlorhydrate d'hydroxylamine, ajouté audit diester sulfosuccinate ou formé *in situ.*

28. Procédé de synthèse, qui comprend les étapes de :
a) estérification d'un composé sulfo-succinimide pour former un premier produit diester, et
b) **caractérisé de plus par** la cyclisation dudit premier produit diester pour former un sulfo-N-hydroxysuccinimide.

29. Procédé de synthèse comprenant l'étape de cyclisation d'un diester d'acide sulfosuccinique pour former un sulfo-N-hydroxy succinimide.

30. Procédé suivant la revendication 29, dans lequel ladite cyclisation est effectuée en présence d'hydroxylamine.

31. Procédé suivant la revendication 30, dans lequel ladite hydroxylamine est le chlorhydrate d'hydroxylamine.
